# EUROPEAN PATENT SPECIFICATION

(11) **EP 2 825 279 B1**
(45) Date of publication and mention of the grant of the patent: **27.05.2020**
(21) Application number: 13760840.2
(22) Date of filing: 15.03.2013
(51) Int. Cl.: B01D 17/04, C02F 1/36, C02F 1/40, B01D 43/00

(54) **ACOUSTOPHORETIC MULTI-COMPONENT SEPARATION TECHNOLOGY PLATFORM**
MEHRKOMPONENTIGE TECHNOLOGIEPLATTFORM FÜR AKUSTOPHORETISCHE TRENNUNG
PLATEFORME DE TECHNOLOGIE DE SÉPARATION MULTI-COMPOSANT ACOUSTOPHORÉTIQUE

(30) Priority: 15.03.2012 US 201261611159 P; 15.03.2012 US 201261611240 P; 21.01.2013 US 201361754792 P
(43) Date of publication of application: 21.01.2015
(73) Proprietor: Flodesign Sonics Inc., Wilbraham, MA 01095 (US)
(72) Inventor: LIPKENS, Bart, Hampden, Massachusetts 01036 (US); DIONNE, Jason, Simsbury, Connecticut 06070 (US); KENNEDY, III, Thomas J., Wilbraham, Massachusetts 01095 (US); MASI, Louis, Wilbraham, MA 01095 (US); KOWALSKI, III, Stanley, Wilbraham, Massachusetts 01095 (US)
(74) Representative: Kador & Partner PartG mbB
(86) International application number: PCT/US2013/032705
(87) International publication number: WO 2013/138797

(56) References cited:
- WO-A1-98/50133
- WO-A1-2010/024753
- US-A- 2 473 971
- US-A- 4 173 725
- US-A- 4 204 096
- US-A- 4 983 189
- US-A- 5 371 429
- US-A- 5 626 767
- US-A1- 2003 015 035
- US-A1- 2011 024 335
- US-A1- 2011 123 392
- US-A1- 2011 154 890

## Description

### BACKGROUND

The present application relates to the acoustophoretic separation technology.

Acoustophoresis is the separation of particles using high intensity sound waves. It has long been known that high intensity standing waves of sound can exert forces on particles. A standing wave has a pressure profile which appears to "stand" still in time. The pressure profile in a standing wave varies from areas of high pressure (nodes) to areas of low pressure (anti-nodes). Standing waves are produced in acoustic resonators. Common examples of acoustic resonators include many musical wind instruments such as organ pipes, flutes, clarinets, and horns.

Efficient separation technologies for multi-component liquid streams that eliminate any waste and reduce the required energy, thereby promoting a sustainable environment, are needed.

### BRIEF DESCRIPTION

The present disclosure relates to systems and devices for acoustophoresis on a large scale. The devices use an ultrasonic transducer as described herein. The transducer is driven at frequencies that produce multiple standing waves.

In some embodiments, an apparatus including a flow chamber with an inlet and an outlet through which is flowed a mixture of a host fluid and at least one of a second fluid and a particulate is disclosed. An ultrasonic transducer embedded in a wall of said flow chamber or located outside the flow chamber wall is driven by an oscillating, periodic, or pulsed voltage signal of ultrasonic frequencies which drives the transducer in a higher order mode of vibration to create standing waves in the flow channel. The transducer includes a ceramic crystal. A reflector is located on the wall on the opposite side of the flow chamber from the transducer.

In other embodiments, a method of separating a host fluid from at least one of a second fluid and a particulate is disclosed. The method comprises flowing the host fluid into a flow chamber having a resonator and a collection pocket and driving a transducer with an oscillating, periodic, or pulsed voltage signal to create standing waves in the resonator and collect the at least one of the second fluid and particulate in the collection pocket.

In yet other embodiments, an apparatus comprises a flow chamber with an inlet and an outlet through which is flowed a mixture of a host fluid and at least one of a second fluid and a particulate. A plurality of ultrasonic transducers are embedded in a wall of said flow chamber or located outside the flow chamber wall. The transducers each include a ceramic crystal driven by an oscillating, periodic, or pulsed voltage signal of ultrasonic frequencies which drives the transducers in a higher order mode of vibration to create standing waves in the flow channel. A reflector is located on the wall on the opposite side of the flow chamber from the transducers.

These and other non-limiting characteristics are more particularly described below.

### BRIEF DESCRIPTION OF THE DRAWINGS

The following is a brief description of the drawings, which are presented for the purposes of illustrating the exemplary embodiments disclosed herein and not for the purposes of limiting the same.
**Figure 1** shows an acoustophoretic separator having one transducer.
**Figure 2** is a diagram illustrating the function of an acoustophoretic separator.
**Figure 3** shows an acoustophoretic separator having a plurality of transducers.
**Figure 4A** is a detail view of a diffuser used as an inlet in the separator of **Figure 3****.**
**Figure 4B** is a detail view of an alternate inlet diffuser that can be used with the separator of **Figure 3****.**
**Figure 5** is a cross-sectional diagram of a conventional ultrasonic transducer.
**Figure 6** is a picture of a wear plate of a conventional transducer.
**Figure 7** is a cross-sectional diagram of an ultrasonic transducer of the present disclosure. An air gap is present within the transducer, and no backing layer is present.
**Figure 8** is a computer model of an acoustophoretic separator simulated to generate **Figures 9-17****.**
**Figures 9-17** are simulations of the forces on a particle in an acoustophoretic separator.
**Figure 18** is a photo of a square transducer and a circular transducer for use in an acoustophoretic separator.
**Figure 19** is a graph of impedance amplitude versus frequency as a square transducer is driven at different frequencies.
**Figure 20** illustrates the node configurations for seven of the peak amplitudes of **Figure 19****.**
**Figure 21** is a photo of the nine-node configuration of a transducer.
**Figure 22** is a photo of another multi-nodal configuration of a transducer.
**Figure 23** is a computer simulation of the forces from a transducer.
**Figures 24** and **25** show transducer array configurations.
**Figure 26** shows an acoustophoretic separator for separating buoyant materials for use with the transducers of **Figures 21** and **22****.**
**Figure 27** is a computer simulation of the forces from an array of transducers.
**Figure 28** is a photo showing the nodes of an array of transducers.
**Figure 29** is a photo showing the nodes of an array of transducers.
**Figure 30** is a computer simulation of the forces from an array of transducers.

### DETAILED DESCRIPTION

The present disclosure may be understood more readily by reference to the following detailed description of desired embodiments and the examples included therein. In the following specification and the claims which follow, reference will be made to a number of terms which shall be defined to have the following meanings.

The singular forms "a," "an," and "the" include plural referents unless the context clearly dictates otherwise.

As used in the specification and in the claims, the term "comprising" may include the embodiments "consisting of" and "consisting essentially of."

Numerical values should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 grams to 10 grams" is inclusive of the endpoints, 2 grams and 10 grams, and all the intermediate values). The endpoints of the ranges and any values disclosed herein are not limited to the precise range or value; they are sufficiently imprecise to include values approximating these ranges and/or values.

As used herein, approximating language may be applied to modify any quantitative representation that may vary without resulting in a change in the basic function to which it is related. Accordingly, a value modified by a term or terms, such as "about" and "substantially," may not be limited to the precise value specified, in some cases. In at least some instances, the approximating language may correspond to the precision of an instrument for measuring the value. The modifier "about" should also be considered as disclosing the range defined by the absolute values of the two endpoints. For example, the expression "from about 2 to about 4" also discloses the range "from 2 to 4."

Efficient separation technologies for multi-component liquid streams that eliminate any waste and reduce the required energy, and therefore promote a sustainable environment, are needed. Large volume flow rate acoustophoretic phase separator technology using ultrasonic standing waves provides the benefit of having no consumables, no generated waste, and a low cost of energy. The technology is efficient at removal of particles of greatly varying sizes, including separation of micron and submicron sized particles. Examples of acoustic filters/collectors utilizing acoustophoresis can be found in commonly owned U.S. Patent Applications US2011278218, US2012325727 and US2012328477.

The platform technology described herein provides an innovative solution that includes a large volume flow rate acoustophoretic phase separator based on ultrasonic standing waves with the benefit of having no consumables, no generated waste, and a low cost of energy. Acoustophoresis is a low-power, no-pressure-drop, no-clog, solid-state approach to particle removal from fluid dispersions: i.e., it is used to achieve separations that are more typically performed with porous filters, but it has none of the disadvantages of filters. In particular, the present disclosure provides systems that operate at the macro-scale for separations in flowing systems with high flow rates. The acoustic resonator is designed to create a high intensity three dimensional ultrasonic standing wave that results in an acoustic radiation force that is larger than the combined effects of fluid drag and buoyancy, and is therefore able to trap, i.e., hold stationary, the suspended phase. The present systems have the ability to create ultrasonic standing wave fields that can trap particles in flow fields with linear velocity exceeding 1 cm/s. This technology offers a green and sustainable alternative for separation of secondary phases with a significant reduction in cost of energy. Excellent particle separation efficiencies have been demonstrated for particle sizes as small as one micron.

The acoustophoretic separation technology employs ultrasonic standing waves to trap, i.e., hold stationary, secondary phase particles in a host fluid stream. This is an important distinction from previous approaches where particle trajectories were merely altered by the effect of the acoustic radiation force. The scattering of the acoustic field off the particles results in a three dimensional acoustic radiation force, which acts as a three-dimensional trapping field. The acoustic radiation force is proportional to the particle volume (e.g. the cube of the radius). It is proportional to frequency and the acoustic contrast factor. It also scales with acoustic energy (e.g. the square of the acoustic pressure amplitude). The sinusoidal spatial variation of the force is what drives the particles to the stable positions of the standing waves. When the acoustic radiation force exerted on the particles is stronger than the combined effect of fluid drag force and buoyancy/gravitational force, the particle is trapped within the acoustic standing wave field. The action of the acoustic forces on the trapped particles results in concentration, agglomeration and/or coalescence of particles and droplets. Heavier-than-water (i.e. denser than water) particles are separated through enhanced gravitational settling, and lighter-than-water particles are separated through enhanced buoyancy.

Efficient and economic particle separation processes can be useful in many areas of energy generation, e.g., producing water, hydro-fracking, and bio-fuels, e.g, harvesting and dewatering. Acoustophoretic technology can be used to target accelerated capture of bacterial spores in water, oil-recovery, and dewatering of bio-oil derived from micro-algae. Current technology used in the oil recovery field does not perform well in recovery of small, i.e., less than 20 micron, oil droplets. However, the acoustophoretic systems described herein can enhance the capture and coalescence of small oil droplets, thereby shifting the particle size distribution resulting in an overall increased oil capture. To be useful, it is generally necessary to demonstrate large flow rates at a level of 15 litres per minute (4 gallons per minute (GPM), the conversion of 1 GPM = 3,7854 litres per minute applies throughout the description). Another goal is the increased capture of oil droplets with a diameter of less than 20 microns.

Acoustophoretic separation can also be used to aid such applications as advanced bio-refining technology to convert low-cost readily available non-food biomass (e.g. municipal solid waste and sewage sludge) into a wide array of chemicals and secondary alcohols that can then be further refined into renewable gasoline, jet fuel, or diesel. A water treatment technology is used to de-water the fermentation broth and isolate valuable organic salts for further processing into fuels. The dewatering process is currently done through an expensive and inefficient ultra-filtration method that suffers from frequent fouling of the membranes, a relatively low concentration factor, and a high capital and operating expense. Acoustophoretic separation can filter out particles with an incoming particle size distribution that spans more than three orders of magnitude, namely from 600 microns to 0.3 microns, allowing improvements in the concentration of the separated broth with a lower capital and operational expense.

Acoustophoretic separation is also useful for the harvesting, oil-recovery, and dewatering of micro-algae for conversion into bio-oil. Current harvesting, oil recovery, and dewatering technologies for micro-algae suffer from high operational and capital expenses. Current best estimates put the price of a barrel of bio-oil derived from micro-algae at a minimum of $200.00 per barrel. There is a need in the art of micro-algae biofuel for technologies that improve harvesting, oil-recovery, and dewatering steps of this process. Acoustophoretic separation technology meets this need.

Some other applications are in the areas of wastewater treatment, grey water recycling, and water production. Other applications are in the area of life sciences and medical applications, such as the separation of lipids from red blood cells. This can be of critical importance during cardiopulmonary bypass surgery, which involves suctioning shed mediastinal blood. Lipids are unintentionally introduced to the bloodstream when blood is re-transfused to the body. Lipid micro-emboli can travel to the brain and cause various neuro-cognitive disorders. Therefore, there is a need to cleanse the blood. Existing methods are currently inefficient or harmful to red blood cells.

Particular embodiments focus on the capture and growth of sub 20 micron oil droplets. At least 80% of the volume of sub-20-micron droplets are captured and then grown to droplets that are bigger than 20 microns. The process involves the trapping of the oil droplets in the acoustic standing wave, coalescence of many small trapped droplets, and eventually release of the larger droplets when the acoustic trapping force becomes smaller than the buoyancy force.

Advanced multi-physics and multiple length scale computer models and high frequency (MHz), high-power, and high-efficiency ultrasonic drivers with embedded controls have been combined to arrive at new designs of acoustic resonators driven by arrays of piezoelectric transducers, resulting in acoustophoretic separation devices that far surpass current capabilities.

Desirably, such transducers provide a transverse force to accompany the axial force so as to increase the particle trapping capabilities of a acoustophoretic system.

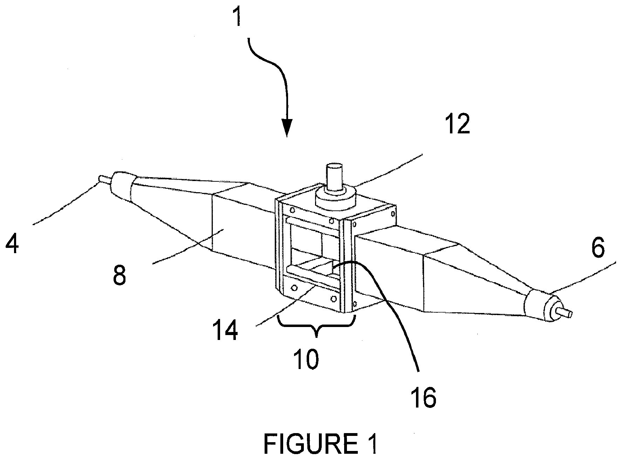

A schematic representation of one embodiment of an acoustophoretic particle separator **1** is shown in **Figure 1****.** A multi-component liquid stream (e.g. water or other fluid) enters the inlet **4** and separated fluid exits at the opposite end via outlet **6.** It should be noted that this liquid stream is usually under pressure when flowing through the separator. The particle separator **1** has a longitudinal flow channel **8** that carries the multi-component liquid stream and passes through a resonator **10.** The resonator **10** includes a transducer **12** or, in some embodiments, an array of transducers, which acts as an excitation source of acoustic waves. The acoustic resonator **10** has a reflector **14**, which is located on the wall opposite the transducer **12.** A collection pocket **16** collects impurities, and is also located opposite the transducer. As defined herein, impurities includes particles or fluids distinct from the host fluid. The acoustic resonator **10** is designed to maintain a high intensity three-dimensional acoustic standing wave. The system is driven by a function generator and amplifier (not shown). The system performance is monitored and controlled by a computer.

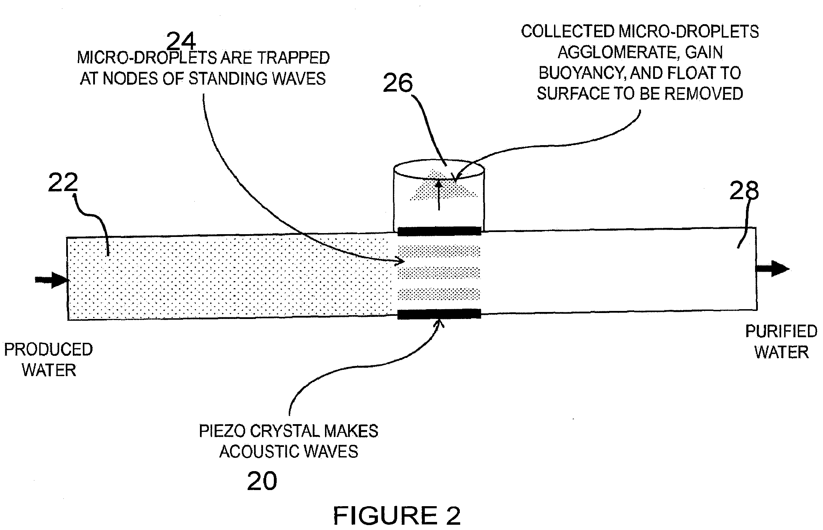

A diagrammatic representation of an embodiment for removing oil or other lighter-than-water material is shown in **Figure 2****.** Excitation frequencies typically in the range from 100s of kHz to several MHz are applied by transducer **20.** Microdroplets **22** are trapped at standing waves **24**, agglomerate, and, in the case of buoyant material, float to the surface and are discharged via an effluent outlet **26.** Purified water is discharged at outlet **28.** The acoustophoretic separation technology can accomplish multi-component particle separation without any fouling at a much reduced cost.

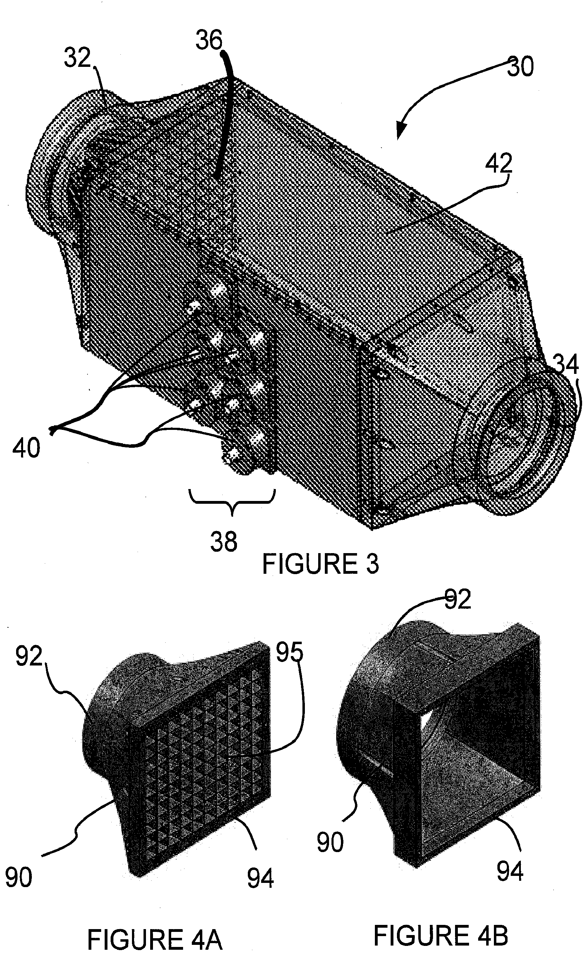

**Figure 3** shows another embodiment of an acoustophoretic particle separator **30.** Like acoustophoretic separator **1**, acoustophoretic separator **30** has an inlet **32** and an outlet **34.** The inlet **32** is fitted with a nozzle or diffuser **90** having a honeycomb 95 to facilitate the development of plug flow. The acoustophoretic separator **30** has an array **38** of transducers **40**, in this case six transducers all arranged on the same wall. The transducers are arranged so that they cover the entire cross-section of the flowpath. The acoustophoretic separation system of **Figure 3** has, in certain embodiments, a square cross section of 15.24 cm x 15.24 cm (6 inches x 6 inches, the conversion of 1 inch (1") = 2.54 cm applies throughout the description) which operates at flow rates of up to 11 litres per minute (3 gallons per minute (GPM)), or a linear velocity of 8 mm/sec. The transducers **40** are six PZT-8 (Lead Zirconate Titanate) transducers with a 2.54 cm (1 inch) diameter and a nominal 2 MHz resonance frequency. Each transducer consumes about 28 W of power for droplet trapping at a flow rate of 11 litres per minute (3 GPM). This translates in an energy cost of 0.25 kW hr/ m³. This is an indication of the very low cost of energy of this technology. Desirably, each transducer is powered and controlled by its own amplifier.

**Figure 4A** and **Figure 4B** show two different diffusers that can be used at the inlet of the acoustophoretic separator. The diffuser **90** has an entrance **92** (here with a circular shape) and an exit **94** (here with a square shape). The diffuser of **Figure 4A** is illustrated in **Figure 3. Figure 4A** includes a grid or honeycomb **95,** whereas **Figure 4B** does not. The grid helps ensure uniform flow.

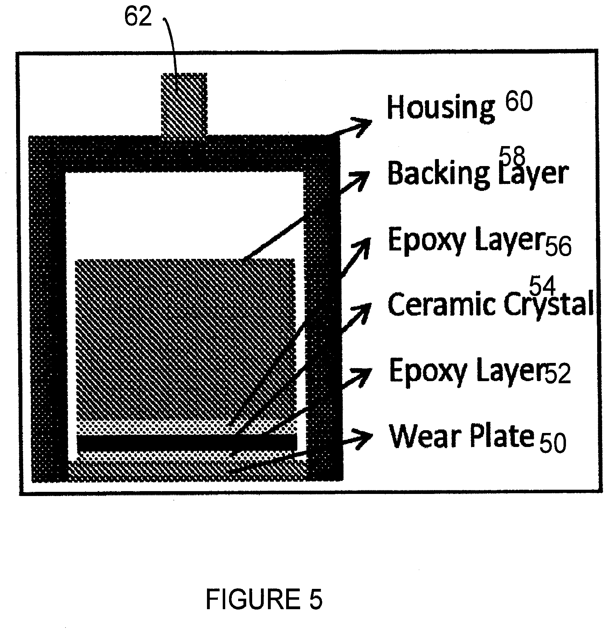

**Figure 5** is a cross-sectional diagram of a conventional ultrasonic transducer. This transducer has a wear plate **50** at a bottom end, epoxy layer **52,** ceramic crystal **54** (made of, e.g. PZT), an epoxy layer **56,** and a backing layer **58.** The epoxy layer **56** attaches backing layer **58** to the crystal **54.** The entire assembly is contained in a housing **60** which may be made out of, for example, aluminum. A connector **62** provides connection for wires to pass through the housing and connect to leads (not shown) which attach to the crystal **54.**

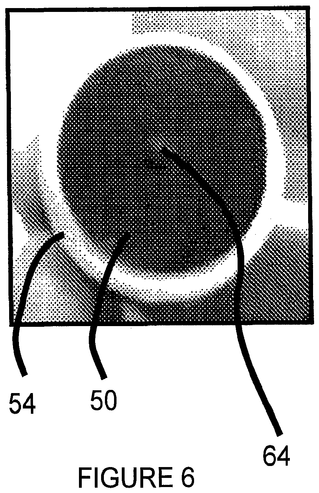

**Figure 6** is a photo of a wear plate **50** with a bubble **64** where the wear plate has pulled away from the ceramic crystal surface due to the oscillating pressure.

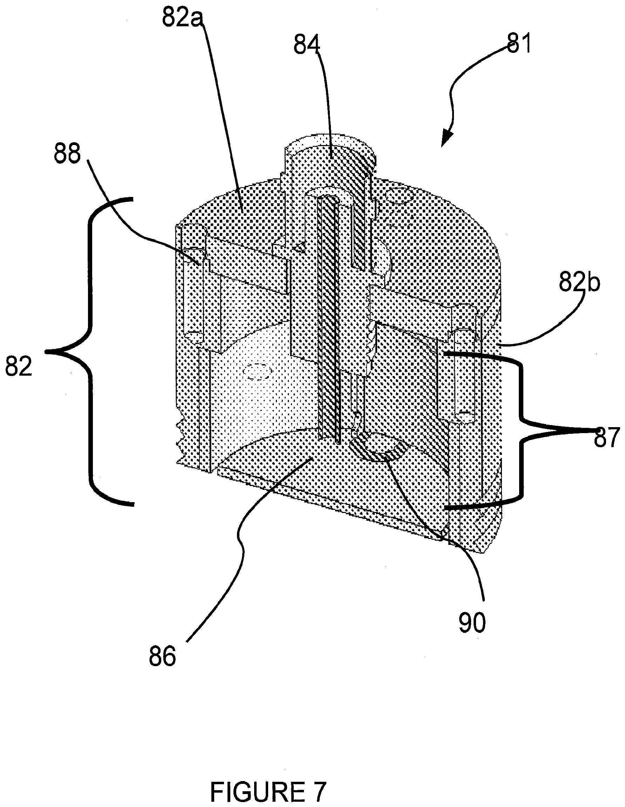

**Figure 7** is a cross-sectional view of an ultrasonic transducer **81** of the present disclosure, which can be used with the acoustophoretic separators of **Figure 1** and **Figure 3****.** Transducer **81** has an aluminum housing **82.** A PZT crystal **86** defines the bottom end of the transducer, and is exposed from the exterior of the housing. The crystal is supported on its perimeter by the housing.

Screws (not shown) attach an aluminum top plate **82a** of the housing to the body **82b** of the housing via threads **88.** The top plate includes a connector **84** to pass power to the PZT crystal **86.** Electrical power is provided to the PZT crystal **86** by electrical lead **90.** Note that the crystal **86** has no backing layer as is present in **Figure 5****.** Put another way, there is an air gap **87** in the transducer between aluminum top plate **82a** and the crystal **86.** A minimal backing may be provided in some embodiments.

The transducer design can affect performance of the system. A typical transducer is a layered structure with the ceramic crystal bonded to a backing layer and a wear plate. Because the transducer is loaded with the high mechanical impedance presented by the standing wave, the traditional design guidelines for wear plates, e.g., half or quarter wavelength thickness, and manufacturing methods may not be appropriate. Rather, in one embodiment of the present disclosure the transducers, there is no wear plate or backing, allowing the crystal to vibrate with a high Q-factor. The vibrating ceramic crystal/disk is directly exposed to the fluid flowing through the flow chamber.

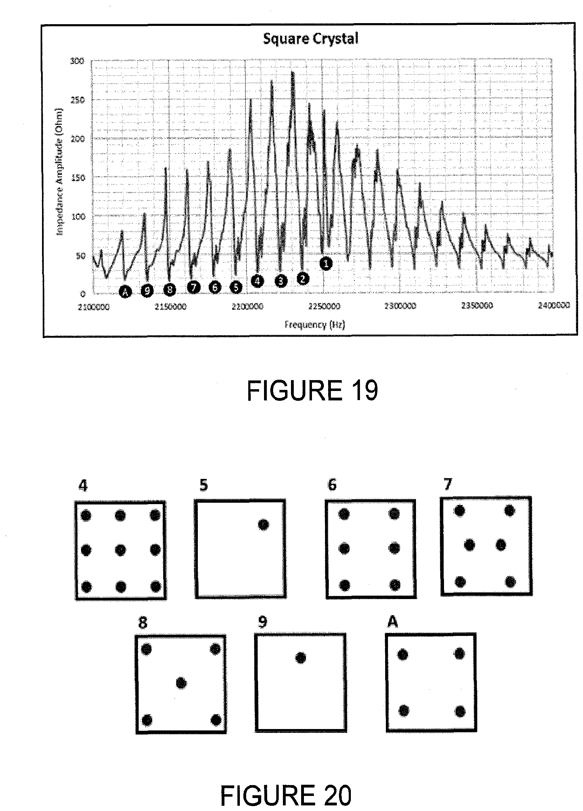

Removing the backing (e.g. making the crystal air backed) also permits the ceramic crystal to obtain higher order modes of vibration (e.g. higher order modal displacement). In a transducer having a crystal with a backing, the crystal vibrates with a uniform displacement, like a piston. Removing the backing allows the crystal to vibrate in a non-uniform displacement mode. The higher order the mode shape of the crystal, the more nodal lines the crystal has. The higher order modal displacement of the crystal creates more trapping lines, although the correlation of trapping line to node is not necessarily one to one, and driving the crystal at a higher frequency will not necessarily produce more trapping lines. See the discussion below with respect to Figures 19-22.

In some embodiments, the crystal may have a backing that minimally affects the Q-factor of the crystal (e.g. less than 5%). The backing may be made of a substantially acoustically transparent material such as balsa wood or cork which allows the crystal to vibrate in a higher order mode shape and maintains a high Q-factor while still providing some mechanical support for the crystal. In another embodiment, the backing may be a lattice work that follows the nodes of the vibrating crystal in a particular higher order vibration mode, providing support at node locations while allowing the rest of the crystal to vibrate freely. The goal of the lattice work or acoustically transparent material is to provide support without lowering the Q-factor of the crystal.

Placing the crystal in direct contact with the fluid also contributes to the high Q-factor by avoiding the dampening and energy absorption effects of the wear plate. Other embodiments may have wear plates or a wear surface to prevent the PZT, which contains lead, contacting the host fluid. This may be desirable in, for example, biological applications such as separating blood. Such applications might use a wear layer such as chrome, electrolytic nickel, or electroless nickel. Chemical vapor deposition could also be used to apply a layer of poly(p-xylxyene) (e.g. Parylene) or other polymer. Organic and biocompatible coatings such as silicone or polyurethane are also contemplated as a wear surface.

In the present systems, the system is operated at a voltage such that the particles are trapped in the ultrasonic standing wave, i.e., remain in a stationary position. The particles are collected in along well defined trapping lines, separated by half a wavelength. Within each nodal plane, the particles are trapped in the minima of the acoustic radiation potential. The axial component of the acoustic radiation force drives the particles, with a positive contrast factor, to the pressure nodal planes, whereas particles with a negative contrast factor are driven to the pressure anti-nodal planes. The radial or lateral component of the acoustic radiation force is the force that traps the particle. In systems using typical transducers, the radial or lateral component of the acoustic radiation force is typically several orders of magnitude smaller than the axial component of the acoustic radiation force. On the contrary, the lateral force in separators 1 and 30 can be significant, on the same order of magnitude as the axial force component, and is sufficient to overcome the fluid drag force at linear velocities of up to 1 cm/s. As discussed above, the lateral force can be increased by driving the transducer in higher order mode shapes, as opposed to a form of vibration where the crystal effectively moves as a piston having a uniform displacement. These higher order modes of vibration are similar to the vibration of a membrane in drum modes such as modes (1,1), (1,2), (2,1), (2,2), (2, 3), or (m, n), where m and n are 1 or greater. The acoustic pressure is proportional to the driving voltage of the transducer. The electrical power is proportional to the square of the voltage.

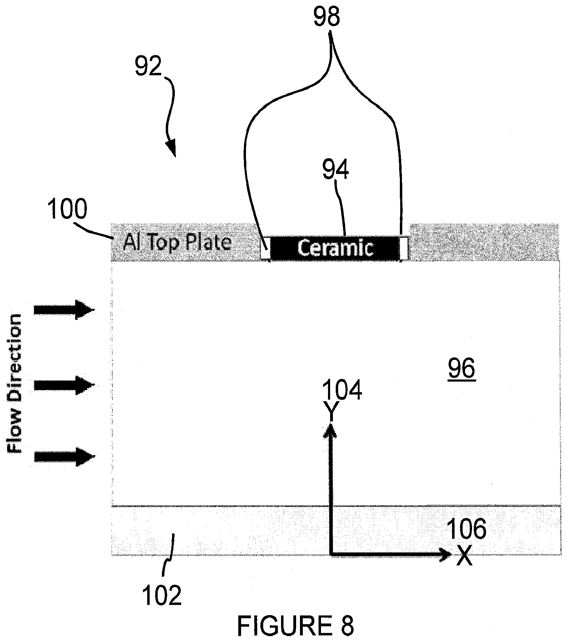

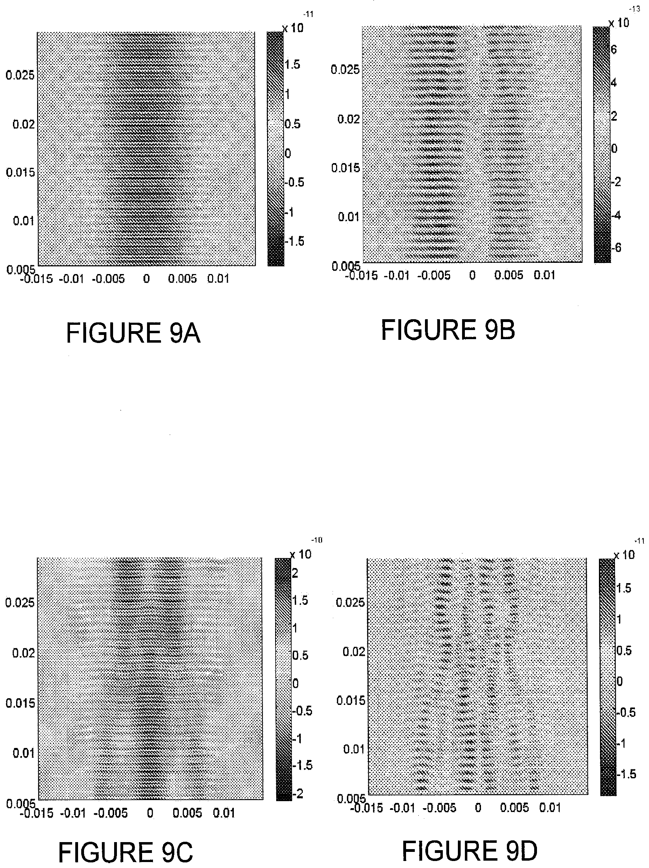

**Figure 8** is a computer model of an acoustophoretic separator 92 simulated to produce **Figures 9-17**. The piezo ceramic crystal **94** is in direct contact with the fluid in the water channel **96.** A layer of silicon **98** is between the crystal **94** and the aluminum top plate **100.** A reflector **102** reflects the waves to create standing waves. The reflector is made of a high acoustic impedance material such as steel or tungsten, providing good reflection. For reference, the Y-axis **104** will be referred to as the axial direction. The X-axis **106** will be referred to as the radial or lateral direction. The acoustic pressure and velocity models were calculated in COMSOL including piezo-electric models of the PZT transducer, linear elastic models of the surrounding structure (e.g. reflector plate and walls), and a linear acoustic model of the waves in the water column. The acoustic pressure and velocity was exported as data to MATLAB. The radiation force acting on a suspended particle was calculated in MATLAB using Gor'kov's formulation. The particle and fluid material properties, such as density, speed of sound, and particle size, are entered into the program, and used to determine the monopole and dipole scattering contributions. The acoustic radiation force is determined by performing a gradient operation on the field potential U, which is a function of the volume of the particle and the time averaged potential and kinetic energy of the acoustic field.

**Figures 9A-9D** show simulations of the difference in trapping between a single acoustic wave and a multimode acoustic wave. **Figure 9A** shows the axial force associated with a single standing acoustic wave. **Figure 9B** shows the lateral force due to a single standing acoustic wave. **Figures 9C** and **9D** show the axial force and lateral force, respectively, in a multi-mode (higher order vibration modes having multiple nodes) piezoelectric crystal excitation where multiple standing waves are formed. The electrical input is the same as the single mode of **Figures 9A** and **9B****,** but the trapping force (lateral force) is 70 times greater (note the scale to the right in **Figure 9B** compared to **9D**). The figures were generated by a computer modeling simulation of a 1MHz piezo-electric transducer driven by 10 V AC potted in an aluminum top plate in an open water channel terminated by a steel reflector (see **Figure 8****).** The field in **Figures 9A** and **9B** is 960 kHz with a peak pressure of 400 kPa. The field in **Figures 9C** and **9D** is 961 kHz with a peak pressure of 1400 kPa. In addition to higher forces, the 961 kHz field **(****Figures 9C** and **D****)** has more gradients and focal spots.

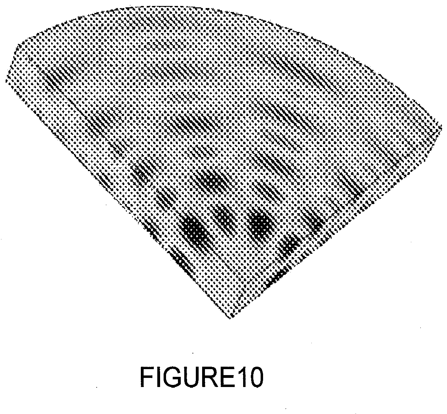

**Figure 10** shows a three dimensional computer generated model of a mode shape calculation for a circular crystal driven at a frequency of 1 MHz.

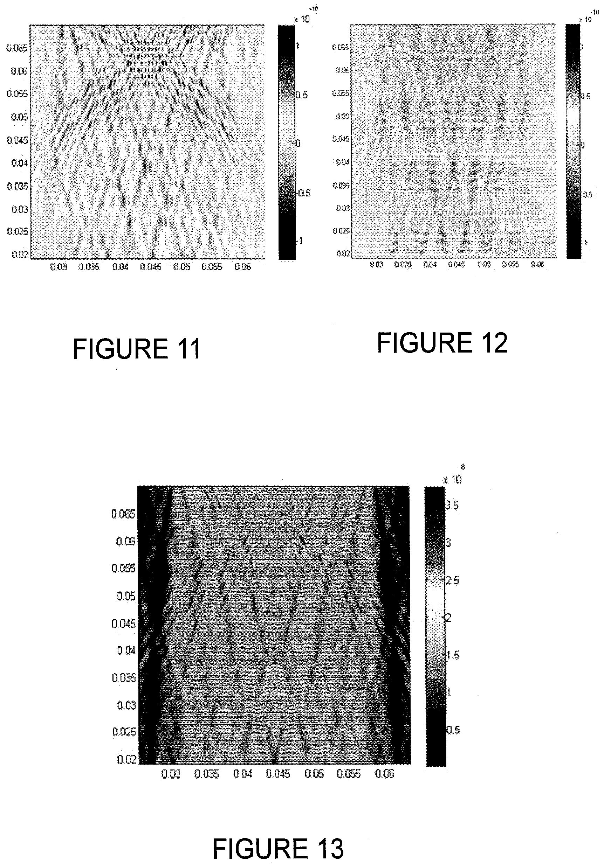

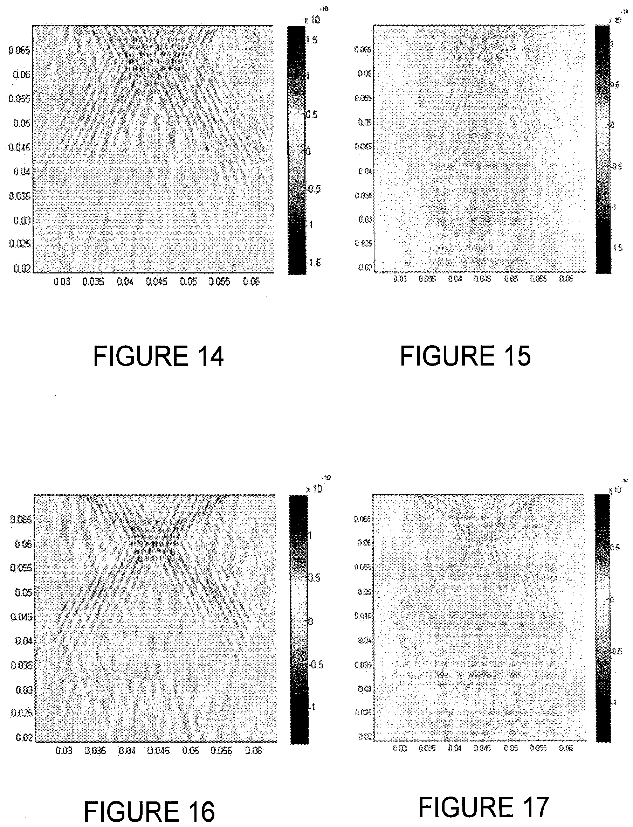

**Figures 11-17** are based on the model of **Figure 8** with a PZT-8 piezo-electric transducer operating at 2 MHz. The transducer is 1" wide and 0.04" thick, potted in an aluminum top plate (0.125" thick) in a 4"x 2" water channel terminated by a steel reflector plate (0.180" thick). The acoustic beam spans a distance of 2". The depth dimension, which is 1", is not included in the 2D model. The transducer is driven at 15V and a frequency sweep calculation is done to identify the various acoustic resonances. The results of the three consecutive acoustic resonance frequencies, i.e., 1.9964 MHz **(****Figures 11, 12,** and **13****),** 2.0106 MHz **(****Figures 14** and **15****),** and 2.025 MHz **(****Figures 16** and **17**), are shown. The acoustic radiation force is calculated for an oil droplet with a radius of 5 micron, a density of 880 kg/m³, and speed of sound of 1700 m/sec. Water is the main fluid with a density of 1000 kg/m³, speed of sound of 1500 m/sec, and dynamic viscosity of 0.001 kg/msec. **Figures 11** shows the lateral (horizontal) acoustic radiation force. **Figure 12** shows the axial (vertical) component for a resonance frequency of 1.9964 MHz. **Figure 13** shows the acoustic pressure amplitude.

**Figures 11** and **12** show that the relative magnitude of the lateral and axial component of the radiation force are very similar, about 1.2e-10 N, indicating that it is possible to create large trapping forces, where the lateral force component is of similar magnitude or higher than the axial component. This is a new result and contradicts typical results mentioned in the literature.

A second result is that the acoustic trapping force magnitude exceeds that of the fluid drag force, for typical flow velocities on the order of mm/s, and it is therefore possible to use this acoustic field to trap the oil droplet. Of course, trapping at higher flow velocities can be obtained by increasing the applied power to the transducer. That is, the acoustic pressure is proportional to the driving voltage of the transducer. The electrical power is proportional to the square of the voltage.

A third result is that at the frequency shown, high trapping forces associated with this particular trapping mode extend across the entire flow channel, thereby enabling capture of oil droplets across the entire channel width. Finally, a comparison of the minima of the acoustic trapping force field, i.e., the locations of the trapped particles, with the observed trapping locations of droplets in the standing wave shows good agreement, indicating that COMSOL modeling is indeed an accurate tool for the prediction of the acoustic trapping of particles. This will be shown in more detail below.

**Figure 14** shows the lateral force component at a resonance frequency of 2.0106 MHz, and **figure 15** shows the axial acoustic radiation force component at a resonance frequency of 2.0106 MHz. **Figures 14** and **15** exhibit higher peak trapping forces than **Figures 11** and **12****.** The lateral acoustic radiation forces exceed the axial radiation force. However, the higher trapping forces are located in the upper part of the flow channel, and do not span the entire depth of the flow channel. It would therefore represent a mode that is effective at trapping particles in the upper portion of the channel, but not necessarily across the entire channel. Again, a comparison with measured trapping patterns indicates the existence of such modes and trapping patterns.

**Figure 16** shows the lateral force component at a resonance frequency of 2.025 MHz, and **figure 17** shows the axial acoustic radiation force component at a resonance frequency of 2.025 MHz. The acoustic field changes drastically at each acoustic resonance frequency, and therefore careful tuning of the system is critical. At a minimum, 2D models are necessary for accurate prediction of the acoustic trapping forces.

2D axisymmetric models were developed to calculate the trapping forces for circular transducers. The models were used to predict acoustic trapping forces on particles, which can then be used to predict particle trajectories in combination with the action of fluid drag and buoyancy forces. The models clearly show that it is possible to generate lateral acoustic trapping forces necessary to trap particles and overcome the effects of buoyancy and fluid drag. The models also show that circular transducers do not provide for large trapping forces across the entire volume of the standing wave created by the transducer, indicating that circular transducers only yield high trapping forces near the center of the ultrasonic standing wave generated by the transducer, but provide much smaller trapping forces toward the edges of the standing wave. This further indicates that the circular transducer only provides limited trapping for a small section of the fluid flow that would flow across the standing wave of the circular transducer, and no trapping near the edges of the standing wave.

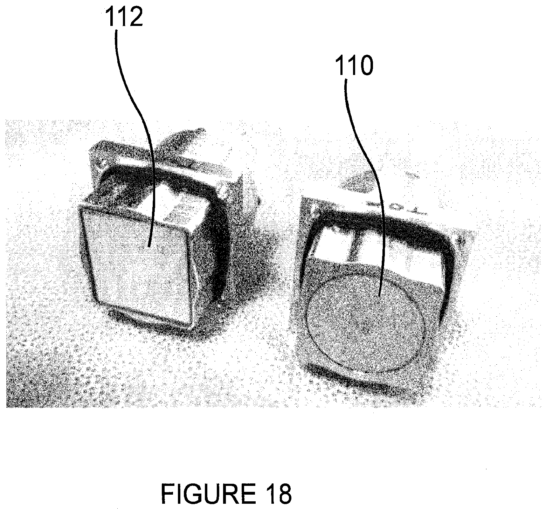

Because the circular transducers do not provide for large trapping forces across the entire volume, the effect of transducer shape on oil separation efficiency was investigated. A 1 "-diameter circular PZT-8 crystal (**Figure 18**, **110**) and a 1"x1" square crystal (**Figure 18****, 112**) were used. Otherwise the experiment was run at identical conditions. Table 1 shows the results.

**Table 1: Results of Investigation of Round and Square Transducer Shape**

| **Transducer Shape** | **Total Power Input (Watts)** | **Flowrate (ml/min)** | **Duration (min)** | **Capture Efficiency (%)** |
|---|---|---|---|---|
| Round | 20 | 500 | 45 | 59% |
| Square | 20 | 500 | 30 | 91% |

The results indicate that the square transducer **112** provides better oil separation efficiencies than the round transducer **110**, explained by the fact that the square transducer **112** provides better coverage of the flow channel with acoustic trapping forces, and that the round transducer only provides strong trapping forces along the centerline of the standing wave, confirming the findings of the numerical simulations.

In addition to the shape of the transducer, the shape of the mode of the transducer (in what shape the transducer is vibrating) affects oil separation efficiency. Producing more nodes provides more places for oil to be trapped. **Figure 19** shows the measured electrical impedance amplitude of the transducer as a function of frequency in the vicinity of the 2.2 MHz transducer resonance. The minima in the transducer impedance correspond to acoustic resonances of the water column and represent potential frequencies for operation. Numerical modeling has indicated that the transducer displacement profile varies significantly at these acoustic resonance frequencies, and thereby directly affects the acoustic standing wave and resulting trapping force. The transducer displacement mode shape varies from a single half wavelength mode to a three half wavelength mode shape. Higher order transducer modal displacement patterns result in higher trapping forces and multiple stable trapping locations for the captured oil droplets. A single half wavelength mode results in one line of trapped droplets, whereas a three half wavelength mode results in three parallel lines of trapped droplets across the fluid channel.

To investigate the effect of transducer mode shape on acoustic trapping force and oil separation efficiencies, an experiment was repeated ten times, with all conditions identical except for the excitation frequency. Ten consecutive acoustic resonance frequencies, indicated by circled numbers 1-9 and letter A on **Figure 19****,** were used as excitation frequencies. The conditions were experiment duration of 30 min, a 1000 ppm oil concentration, a flow rate of 500 ml/min, and an applied power of 20W.

As the emulsion passed by the transducer, the trapping nodal lines were observed and characterized. The characterization involved the observation and pattern of the number of nodal trapping lines across the fluid channel, as shown in **Figure 20****,** for seven of the ten resonance frequencies identified in **Figure 19****.**

The effect of excitation frequency clearly determines the number of nodal trapping lines, which vary from a single trapping line at the excitation frequency of acoustic resonance 5 and 9, to nine trapping nodal lines for acoustic resonance frequency 4. At other excitation frequencies four or five nodal trapping lines are observed. These experimentally observed results confirm the results expected from the differences when **Figures 9A** and **9B** are compared to **Figures 9C** and **9D****.** Different modes of vibration of the transducer can produce different (more) nodes of the standing waves, with more nodes generally creating higher trapping forces.

Table 2 summarizes the findings from an oil trapping experiment using a system similar to **Figure 1****.** An important conclusion is that the oil separation efficiency of the acoustic separator is directly related to the mode shape of the transducer. Higher order modal displacements generate larger acoustic trapping forces and more trapping nodal lines resulting in better efficiencies. A second conclusion, useful for scaling studies, is that the tests indicate that capturing 5 micron oil droplets at 500 ml/min requires 10 Watts of power per square-inch of transducer area per 1" of acoustic beam span. The main dissipation is that of thermo-viscous absorption in the bulk volume of the acoustic standing wave. The cost of energy associated with this flow rate is 0.667 kWh per cubic meter.

**Table 2: Trapping Pattern Capture Efficiency Study**

| **Resonance Peak Location** | **Total Power Input (Watts)** | **# of Trapping Nodes** | **Flowrate (ml/min)** | **Duration (min)** | **Capture Efficiency (%)** |
|---|---|---|---|---|---|
| **4** | 20 | **9** | 500 | 30 | 91% |
| **8** | 20 | **5** | 500 | 30 | 58% |
| **A** | 20 | **4** | 500 | 30 | 58% |
| **9** | 20 | **2** | 500 | 30 | 37% |

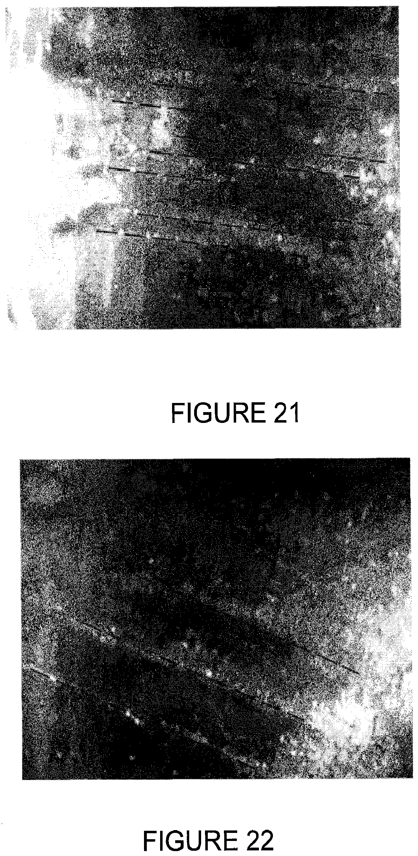

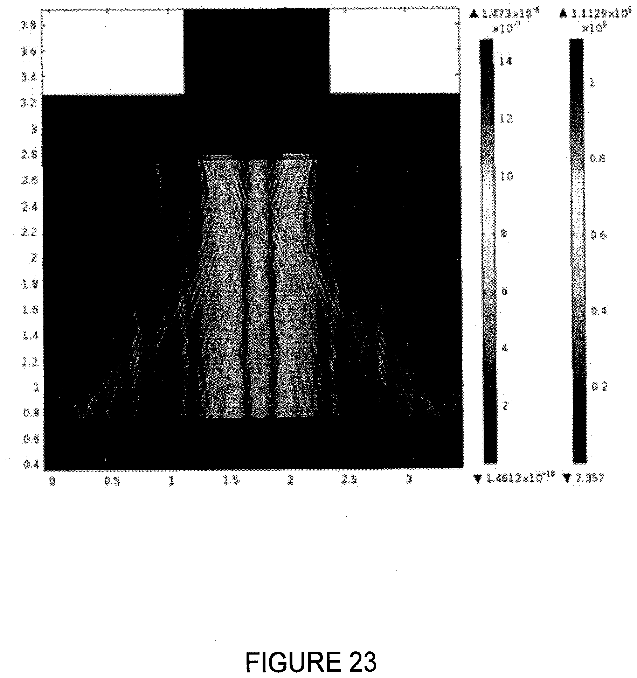

**Figures 21** and **22** show photos of the trapped oil droplets in the nine trapping nodal line pattern. Dashed lines are superimposed over the nodal lines. **Figure** 23 shows the pressure field, calculated in COMSOL that matches the 9 trapping nodal line pattern. The numerical model is a two-dimensional model; and therefore only three trapping columns are observed. Two more sets of three trapping columns exist in the third dimension perpendicular to the plane of the 2D model of **Figure 21** and **22**. This comparison indicates that the numerical model is accurate in predicting the nature of the ultrasonic standing wave and the resulting trapping forces, again confirming the results expected from the differences when **Figures 9A** and **9B** are compared to **Figures 9C** and **9D****.**

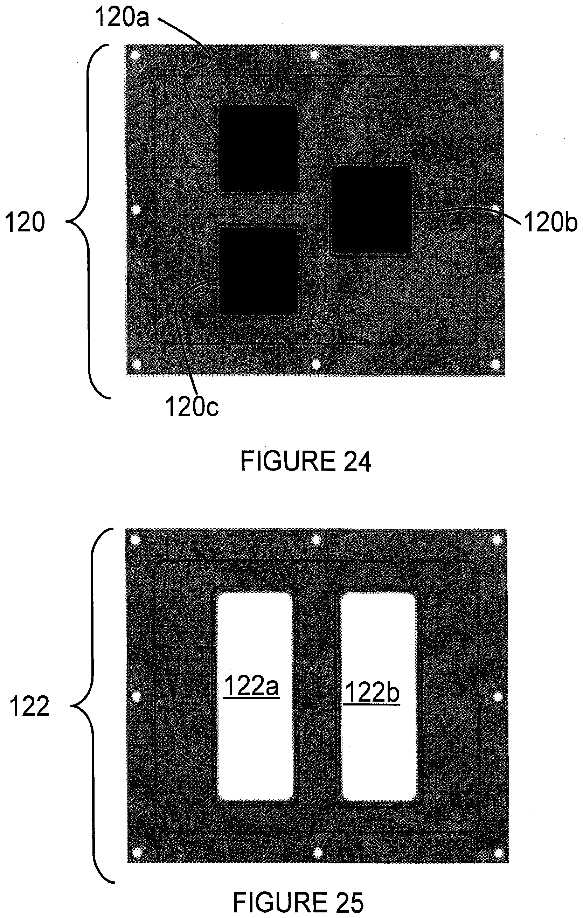

In larger systems, different transducer arrangements are feasible. **Figure 24** shows a transducer array 120 including three square 1"x1" crystals **120a, 120b, 120c.** Two squares are parallel to each other, and the third square is offset to form a triangular pattern. **Figure 25** shows a transducer array 122 including two rectangular 1" x 2.5" crystals **122a**, **122b** arranged with their long axes parallel to each other. Power dissipation per transducer was 10 W per 1"x1" transducer cross-sectional area and per inch of acoustic standing wave span in order to get sufficient acoustic trapping forces. For a 4" span of an intermediate scale system, each 1"x1" square transducer consumes 40 W. The larger 1"x2.5" rectangular transducer uses 100W in an intermediate scale system. The array of three 1"x1" square transducers would consume a total of 120 W and the array of two 1 "x2.5" transducers would consume about 200 W.

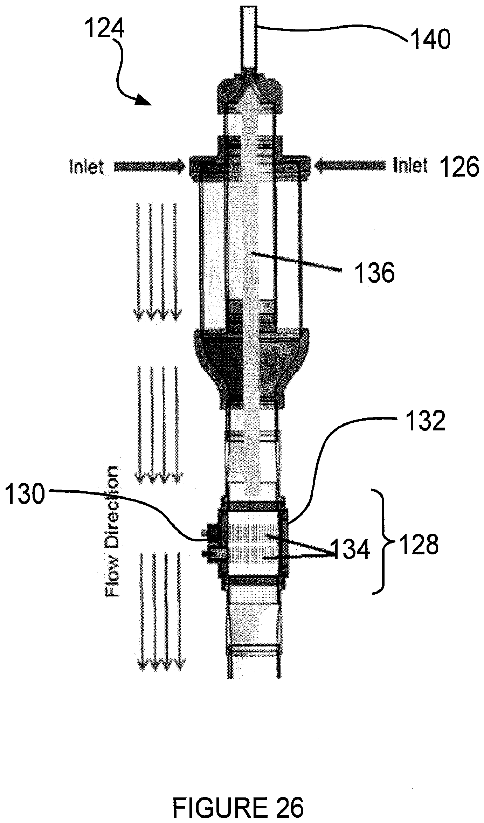

A 4" intermediate scale system **124** for separating a host fluid from a buoyant fluid or particulate is shown in **Figure 26**. Host fluid enters inlet **126** and flows down to the separator **128**, which includes a transducer array **130**, and reflector **132.** The separator creates standing waves **134** to agglomerate buoyant fluid or particulate (e.g. oil). The buoyant force **136** carries the buoyant material to the collection chamber **140.**

Transducer array **120** was installed in system **124**, removed, and then transducer array **122** installed. The arrays were operated in parallel such that each transducer was driven by the same voltage signal from the amplifier. The electronic drive circuit consisted of a function generator and a 300W A300 ENI RF amplifier. The results of the testing are shown in Table 3. The first test used only the two of the 1"x1" square transducers or array **120**, oriented parallel to each other, and was run at a flow rate of 1300 ml/min. It resulted in an oil separation efficiency of 88%. The next test involved all three square transducers and a flow rate of 2000 ml/min, and yielded an efficiency of 93%. These results are excellent and demonstrate that the technology is scalable to larger flow channels driven by arrays of transducers. The next set of tests involved the 1"x2.5" rectangular transducer array **122.** For the first test, only one transducer was run and yielded an efficiency of 87%. The second test with both transducers operating yielded an efficiency of 97%. For the 1"x2.5" transducers, the power level that was used was based on operating the transducer at safe levels. For these tests, the cost of energy for the intermediate system is 1 kWh per cubic meter.

**Table 3: Intermediate System Test Results**

| **Transducer Configuration** | **Number of Transducers Active** | **Total Power Input (Watts)** | **Flowrate (ml/min)** | **Duration (min)** | **Capture Efficiency (%)** |
|---|---|---|---|---|---|
| **1"x1" Transducers** | 2 | 80 | 1300 | 15 | 88% |
| **1"x1" Transducers** | 3 | 120 | 2000 | 15 | 93% |
| **1"x2.5"Transducers** | 1 | 100 | 2000 | 8 | 87% |
| **1"x2.5" Transducers** | 2 | 100 | 1000 | 15 | 97% |

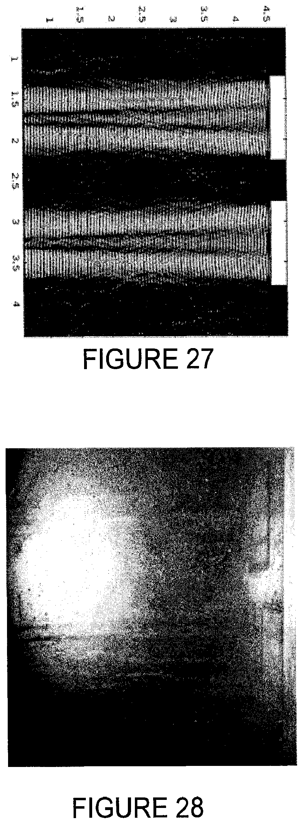

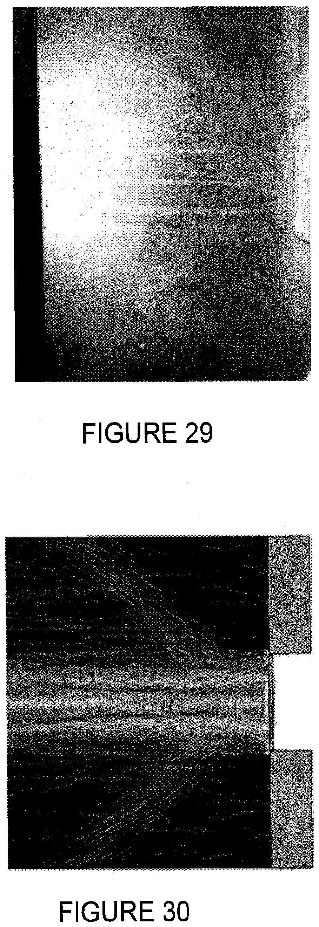

Numerical modeling was also done for the intermediate sized system with a span of 4" for the acoustic standing wave. Multiple transducers were modeled to investigate the coupling effect between transducers. Frequency sweeps were performed and the resonance frequencies for which the acoustic mode shapes couple strongly to the higher order mode shapes of the transducer were identified. The comparisons between numerical and experimental results are excellent and demonstrate the accuracy of the models. **Figure 27** shows the acoustic pressure field of a model with two transducers on the right side. A photograph of the trapped oil droplets in the standing wave is shown in **Figure 28****.** Both experiment and model show identical features. At certain excitation frequencies, oil droplets were trapped in the standing wave well outside the fluid volume defined by the transducer area, indicating an expanded acoustic field with strong trapping forces. **Figure 29** shows a photograph of such trapped oil droplets. **Figure 30** shows an acoustic pressure field model which predicts identical features.

The present disclosure has been described with reference to exemplary embodiments. Obviously, modifications and alterations will occur to others upon reading and understanding the preceding detailed description. It is intended that the present disclosure be construed as including all such modifications and alterations insofar as they come within the scope of the appended claims.

## Claims

1. A method of separating impurities from a host fluid, the method comprising:
providing a flow chamber having a source of acoustic energy and, on an opposing side of the flow chamber, a reflector of acoustic energy;
flowing the host fluid through the flow chamber;
applying the source of acoustic energy to the host fluid to create a three dimensional ultrasonic standing wave, wherein the three dimensional ultrasonic standing wave results in an acoustic radiation force having an axial component and a lateral component that are of the same order of magnitude
wherein the source of acoustic energy is an ultrasonic transducer on a wall of the flow chamber, the transducer including a piezoelectric crystal that defines a side of the transducer, the transducer being driven by an oscillating, periodic, or pulsed voltage signal at an ultrasonic resonance frequency which drives the transducer to create the three dimensional ultrasonic standing wave in the flow chamber.

2. The method of claim 1, wherein the standing wave creates nodal lines and the lateral component traps the impurities in the nodal lines.

3. The method of claim 1, wherein:
the flow chamber has an inlet and an outlet; and
a reflector located on a wall on the opposite side of the flow chamber from the transducer.

4. The method of claim 3, wherein the piezoelectric crystal is driven in a non-uniform displacement mode.

5. The method of claim 4, wherein the piezoelectric crystal is driven in a higher order mode shape having more than one nodal trapping line.

6. The method of claim 3, wherein the piezoelectric crystal of the transducer is directly exposed to fluid flowing through the flow chamber, or wherein the ceramic crystal is made of PZT-8 or wherein the ceramic crystal is square.

7. The method of claim 3, wherein the transducer has a housing containing the piezoelectric crystal.

8. The method of claim 7, wherein the housing includes a top and an air gap, the air gap being disposed between the top and the piezoelectric crystal.

9. The method of claim 8, wherein the piezoelectric crystal does not have a backing layer.

10. The method of claim 3, wherein the flow chamber has a collection pocket in a wall of the flow chamber, or wherein the flow chamber further includes a diffuser at the inlet.

11. The method according to any one of the preceding claims, wherein the impurities include particles or fluids distinct from the host fluid.

12. The method of claim 3, wherein the piezoelectric crystal is backed by balsa wood or cork.

13. The method of claim 2, wherein the impurities trapped in the nodal lines coalesce or agglomerate such that heavier than water impurities are separated through enhanced gravitational settling and lighter than water particles are separated through enhanced buoyancy.

## Patentansprüche

1. Verfahren zum Trennen von Verunreinigungen von einem Host-Fluid, wobei das Verfahren Folgendes umfasst:
Vorsehen einer Strömungskammer, die eine Quelle akustischer Energie und auf einer gegenüberliegenden Seite der Strömungskammer einen Reflektor akustischer Energie aufweist;
Zulassen des Strömens des Hosts-Fluids durch die Strömungskammer;
Anwenden der Quelle akustischer Energie auf das Host-Fluid, um eine dreidimensionale, stehende Ultraschallwelle zu erzeugen, wobei aus der dreidimensionalen, stehenden Ultraschallwelle eine Kraft der akustischen Strahlung resultiert, die eine Axialkomponente und eine Querkomponente derselben Größenordnung aufweist;
wobei die Quelle akustischer Energie ein Ultraschallwandler auf einer Wand der Strömungskammer ist, wobei der Wandler einen piezoelektrischen Kristall enthält, der eine Seite des Wandlers definiert, wobei der Wandler durch ein oszillierendes, periodisches oder gepulstes Spannungssignal mit einer Ultraschallresonanzfrequenz angesteuert wird, das den Wandler derart ansteuert, dass die dreidimensionale, stehende Welle in der Strömungskammer erzeugt wird.

2. Verfahren nach Anspruch 1, wobei die stehende Welle Knotenlinien erzeugt und die Querkomponente die Verunreinigungen in den Knotenlinien fängt.

3. Verfahren nach Anspruch 1, wobei:
die Strömungskammer einen Einlass und einen Auslass aufweist; und
ein Reflektor auf einer Wand auf der dem Wandler gegenüberliegenden Seite der Strömungskammer angeordnet ist.

4. Verfahren nach Anspruch 3, wobei der piezoelektrische Kristall in einem Modus ungleichmäßiger Verschiebungen angesteuert wird.

5. Verfahren nach Anspruch 4, wobei der piezoelektrische Kristall in der Form eines Modus höherer Ordnung angesteuert wird, der mehr als eine Knotenfanglinie aufweist.

6. Verfahren nach Anspruch 3, wobei der piezoelektrische Kristall des Wandlers dem Fluid, das durch die Strömungskammer strömt, direkt ausgesetzt ist oder wobei der Keramikkristall aus PZT-8 hergestellt ist oder wobei der Keramikkristall vierkantig ist.

7. Verfahren nach Anspruch 3, wobei der Wandler ein Gehäuse aufweist, das den piezoelektrischen Kristall enthält.

8. Verfahren nach Anspruch 7, wobei das Gehäuse ein Oberteil und einen Luftspalt enthält, wobei der Luftspalt zwischen dem Oberteil und dem piezoelektrischen Kristall angeordnet ist.

9. Verfahren nach Anspruch 8, wobei der piezoelektrische Kristall keine Verstärkungsschicht aufweist.

10. Verfahren nach Anspruch 3, wobei die Strömungskammer eine Sammeltasche in einer Wand der Strömungskammer aufweist oder wobei die Strömungskammer ferner einen Diffusor am Einlass enthält.

11. Verfahren nach einem der vorhergehenden Ansprüche, wobei die Verunreinigungen Partikel oder Fluids enthalten, die vom Host-Fluid deutlich unterscheidbar sind.

12. Verfahren nach Anspruch 3, wobei der piezoelektrische Kristall durch Balsaholz oder Kork verstärkt ist.

13. Verfahren nach Anspruch 2, wobei sich die an den Knotenlinien gefangenen Verunreinigungen vereinigen oder zusammenballen, derart, dass Verunreinigungen, die schwerer als Wasser sind, durch vergrößerte Gravitationsablagerung getrennt werden und Partikel, die leichter als Wasser sind, durch vergrößerten Auftrieb getrennt werden.

## Revendications

1. Procédé de séparation d'impuretés d'un fluide hôte, le procédé comprenant :
le fait de prévoir une chambre de débit ayant une source d'énergie acoustique et, sur un côté opposé de la chambre de débit, un réflecteur d'énergie acoustique ;
la circulation du fluide hôte dans la chambre de débit ;
l'application de la source d'énergie acoustique au fluide hôte afin de créer une onde stationnaire ultrasonique en trois dimensions, dans lequel l'onde stationnaire ultrasonique en trois dimensions engendre une force de rayonnement acoustique ayant une composante axiale et une composante latérale qui sont du même ordre de grandeur,
dans lequel la source d'énergie acoustique est un transducteur ultrasonique sur une paroi de la chambre de débit, le transducteur comprenant un cristal piézoélectrique qui définit un côté du transducteur, le transducteur étant entraîné par un signal de tension oscillant, périodique ou pulsé à une fréquence de résonance ultrasonique qui entraîne le transducteur afin de créer l'onde stationnaire ultrasonique en trois dimensions dans la chambre de débit.

2. Procédé selon la revendication 1, dans lequel l'onde stationnaire crée des lignes nodales et la composante latérale piège les impuretés dans les lignes nodales.

3. Procédé selon la revendication 1, dans lequel :
la chambre de débit possède une entrée et une sortie ; et
un réflecteur situé sur une paroi du côté opposé de la chambre de débit par rapport au transducteur.

4. Procédé selon la revendication 3, dans lequel le cristal piézoélectrique est entraîné dans un mode de déplacement non-uniforme.

5. Procédé selon la revendication 4, dans lequel le cristal piézoélectrique est entraîné dans une forme de mode d'ordre plus élevé qui possède plusieurs lignes de piégeage nodales.

6. Procédé selon la revendication 3, dans lequel le cristal piézoélectrique du transducteur est directement exposé au fluide qui circule dans la chambre de débit, ou dans lequel le cristal céramique est composé de PZT-8, ou dans lequel le cristal céramique est carré.

7. Procédé selon la revendication 3, dans lequel le transducteur possède une enceinte qui contient le cristal piézoélectrique.

8. Procédé selon la revendication 7, dans lequel l'enceinte comprend une partie supérieure et une lame d'air, la lame d'air étant disposée entre la partie supérieure et le cristal piézoélectrique.

9. Procédé selon la revendication 8, dans lequel le cristal piézoélectrique ne possède pas de couche de support.

10. Procédé selon la revendication 3, dans lequel la chambre de débit possède une poche de collecte dans une paroi de la chambre de débit, ou dans lequel la chambre de débit comprend en outre un diffuseur au niveau de l'entrée.

11. Procédé selon l'une quelconque des revendications précédentes, dans lequel les impuretés comprennent des particules ou des fluides distincts du fluide hôte.

12. Procédé selon la revendication 3, dans lequel le cristal piézoélectrique est supporté par du bois de balsa ou du liège.

13. Procédé selon la revendication 2, dans lequel les impuretés piégées dans les lignes nodales coalescent ou s'agglomèrent de sorte que les impuretés plus lourdes que l'eau soient séparées par un dépôt par gravité amélioré, et les particules plus légères que l'eau soient séparées par une flottabilité améliorée.
